# EUROPÄISCHE PATENTSCHRIFT

(11) **EP 3 391 349 B1**
(45) Veröffentlichungstag und Bekanntmachung des Hinweises auf die Patenterteilung: **10.07.2024**
(21) Anmeldenummer: 16794608.6
(22) Anmeldetag: 10.11.2016
(51) Int. Cl.: G08B 21/06, A61B 5/107, A61B 5/00, G06V 20/59, G06V 40/19, G06V 40/18, A61B 5/11, A61B 5/18

(54) **VERFAHREN UND VORRICHTUNG ZUM KLASSIEREN VON AUGENÖFFNUNGSDATEN ZUMINDEST EINES AUGES EINES INSASSEN EINES FAHRZEUGS UND VERFAHREN UND VORRICHTUNG ZUM ERFASSEN EINER SCHLÄFRIGKEIT UND/ODER EINES SEKUNDENSCHLAFES EINES INSASSEN EINES FAHRZEUGS**
METHOD AND APPARATUS FOR CLASSIFYING EYE OPENING DATA FOR AT LEAST ONE EYE OF AN OCCUPANT OF A VEHICLE, AND METHOD AND APPARATUS FOR SENSING DROWSINESS AND/OR MICROSLEEP IN AN OCCUPANT OF A VEHICLE
PROCÉDÉ ET DISPOSITIF DE CLASSEMENT DE DONNÉES D'YEUX OUVERTS D'AU MOINS UN OEIL D'UN PASSAGER D'UN VÉHICULE AINSI QUE PROCÉDÉ ET DISPOSITIF DE DÉTECTION D'UNE ENVIE DE DORMIR ET/OU D'UN MICROSOMMEIL D'UN PASSAGER D'UN VÉHICULE

(30) Priorität: 14.12.2015 DE 102015225109
(43) Veröffentlichungstag der Anmeldung: 24.10.2018
(73) Patentinhaber: Robert Bosch GmbH, 70442 Stuttgart (DE)
(72) Erfinder: WULF, Felix, 71636 Ludwigsburg (DE); VANDOMMELE, Tjark, 70499 Stuttgart (DE)
(86) Internationale Anmeldenummer: PCT/EP2016/077248
(87) Internationale Veröffentlichungsnummer: WO 2017/102186

(56) Entgegenhaltungen:
- EP-A1- 2 453 427
- WO-A1-2007/145566
- DE-A1- 19 715 519

## Beschreibung

### Stand der Technik

Die Erfindung geht aus von einer Vorrichtung oder einem Verfahren nach Gattung der unabhängigen Ansprüche. Gegenstand der vorliegenden Erfindung ist auch ein Computerprogramm.

Schläfrigkeit und Sekundenschlaf am Steuer führen häufig zu gefährlichen Situationen oder Unfällen. Heutige Schläfrigkeitserkennungssysteme geben eine Warnung aus, wenn der Fahrer einen gewissen Schläfrigkeitsgrenzwert überschreitet. Dann wird im Sichtfeld des Fahrers zum Hinweis auf die Überschreitung des Schläfrigkeitsgrenzwertes ein Symbol, z. B. eine Kaffeetasse, eingeblendet.

Derzeit in Serie befindliche Schläfrigkeitserkennungssysteme schätzen beispielsweise die Müdigkeit des Fahrers indirekt aus dem Fahrverhalten. Ebenfalls sind Systeme bekannt, die aus Daten einer Videokamera den momentanen Öffnungsgrad der Augen erkennen können. Dies geschieht mit entsprechenden Bildverarbeitungsalgorithmen. Dabei wird jeweils für beide Augen ein Augenöffnungsniveau detektiert.

Die WO 2014/031042 A1 beschreibt ein System, das ein Augenöffnungssignal mit vordefinierten modellierten Signalen fittet, um Blinzel-Events zu erkennen und daraus Rückschlüsse auf die Aufmerksamkeit des Fahrers zu ziehen.

Die Druckschrift WO 2007/145566 A1 offenbart ein Verfahren und eine Vorrichtung zur bestimmung und Analyse einer Örtlichkeit von visuellem Interesse.

Die Druckschrift EP 2 453 427 A1 offenbart ein Erkennungsgerät zur Erkennung eines Augenstatus eines Fahrers, ein Verfahren zur Erkennung eines Augenstatus eines Fahrers sowie ein entsprechendes Programm.

Die Druckschrift DE 197 15 519 A1 offenbart ein Gerät zur Schätzung des Schläfrigkeitsgrades des Fahrers eines Fahrzeugs.

Die WO 2006/092022 beschreibt ein System, das zur Erkennung von Blinzel-Events aus dem Augenöffnungssignal eine sogenannte Referenz-Amplitude für "normale" Blinzel-Events verwendet.

In der Literatur werden Zusammenhänge zwischen den Eigenschaften von Blinzel-Events und der Schläfrigkeit bereits diskutiert, vgl. Hargutt: Das Lidschlussverhalten als Indikator für Aufmerksamkeits- und Müdigkeitsprozesse bei Arbeitshandlungen, 2002.

### Offenbarung der Erfindung

Vor diesem Hintergrund werden mit dem hier vorgestellten Ansatz ein Verfahren zum Klassieren von Augenöffnungsdaten zumindest eines Auges eines Insassen eines Fahrzeugs, ein Verfahren zum Erfassen einer Schläfrigkeit und/oder eines Sekundenschlafes eines Insassen eines Fahrzeugs, weiterhin eine Vorrichtung, die diese Verfahren verwendet, sowie schließlich ein entsprechendes Computerprogramm gemäß den Hauptansprüchen vorgestellt. Durch die in den abhängigen Ansprüchen aufgeführten Maßnahmen sind vorteilhafte Weiterbildungen und Verbesserungen der im unabhängigen Anspruch angegebenen Vorrichtung möglich.

Eine Erfassung von Augenöffnungsdaten bzw. eines Augenöffnungssignals in einem gleitenden Zeitfenster und eine anschließende Durchführung einer Clusteranalyse dieser Daten ermöglicht eine Verbesserung der Erkennungsgüte von Blinzelmerkmalen der Augen eines Fahrzeuginsassen.

In einer Weiterbildung beinhaltet das hierin vorgeschlagene Konzept ein verbessertes Verfahren zur Berechnung eines momentanen Augenöffnungsniveaus - kurz AON bzw. EON (engl. Eye Opening Niveau).

Es wird ein Verfahren zum Klassieren von Augenöffnungsdaten zumindest eines Auges eines Insassen eines Fahrzeugs für eine Schläfrigkeitserfassung und/oder Sekundenschlaferfassung des Insassen vorgestellt, wobei das Verfahren die folgenden Schritte aufweist:
Generieren eines ersten Augenöffnungsdatensatzes zu einem ersten Messzeitpunkt in einem gleitenden Zeitfenster, wobei der erste Augenöffnungsdatensatz zumindest einen Messpunkt aufweist, der einen ersten Augenöffnungsgrad und/oder eine erste Augenlidbewegungsgeschwindigkeit und/oder eine erste Augenlidbewegungsbeschleunigung des Auges des Insassen in dem ersten Messzeitpunkt repräsentiert;
Erfassen eines zweiten Augenöffnungsdatensatzes zu einem zweiten Messzeitpunkt in dem gleitenden Zeitfenster, wobei der zweite Augenöffnungsdatensatz zumindest einen Erfassungspunkt aufweist, der einen zweiten Augenöffnungsgrad und/oder eine zweite Augenlidbewegungsgeschwindigkeit und/oder eine zweite Augenlidbewegungsbeschleunigung des Auges des Insassen in dem zweiten Messzeitpunkt repräsentiert; und
Ausführen einer Clusteranalyse unter Verwendung des zumindest einen Messpunktes und des zumindest einen Erfassungspunktes, um zumindest den ersten Augenöffnungsdatensatz und/oder zweiten Augenöffnungsdatensatz einem ersten Datencluster zuzuordnen, um die Augenöffnungsdaten zu klassieren, wobei der erste Datencluster einen Öffnungszustand des Auges des Insassen repräsentiert.

Die Augenöffnungsdaten können Daten eines Augenöffnungsgrades des Auges sowie Daten einer Geschwindigkeit und/oder einer Beschleunigung einer Bewegung der Augenlider des Auges zu einem Zeitpunkt umfassen. Bei dem Augenöffnungsgrad kann es sich um einen im Metern bzw. Zentimetern oder Millimetern zu bestimmenden Abstand vom oberen zum unteren Augenlid des Auges des Insassen zu dem ersten bzw. zweiten Messzeitpunkt handeln. Die Augenöffnungsdaten können in Form eines Signals über mehrere Messzeitpunkte hinweg bereitgestellt werden. Unter dem Klassieren der Augenöffnungsdaten kann eine Zuordnung der Augenöffnungsdaten zu vorbestimmten Kategorien von Zuständen des Auges verstanden werden. Beispielsweise können Zustände des Auges als offen, geschlossen oder in einer Öffnungs- oder Schließphase unterschieden werden. Der erste bzw. zweite Augenöffnungsdatensatz kann einen ermittelten bzw. erfassten und in einem Koordinatensystem darstellbaren Wert eines Zustands des Auges des Insassen repräsentieren. Der erste Augenöffnungsdatensatz kann eine geeignete Kombination aus Daten des ersten Augenöffnungsgrades, der ersten Augenlidbewegungsgeschwindigkeit und der ersten Augenlidbewegungsbeschleunigung des Auges des Insassen umfassen. Gleiches kann analog für den zweiten Augenöffnungsdatensatz gelten. Der Messpunkt kann einen Wert auf einer Koordinatenachse zur Darstellung ersten Augenöffnungsdatensatzes bilden. Entsprechend kann der Erfassungspunkt einen Wert auf einer Koordinatenachse zur Darstellung zweiten Augenöffnungsdatensatzes bilden. Der zweite Messzeitpunkt kann zeitlich hinter dem ersten Messzeitpunkt in einem gleitenden Zeitfenster liegen. Unter der Clusteranalyse ist ein Verfahren zur Entdeckung von Ähnlichkeitsstrukturen in Datenbeständen der Augenöffnungsdatensätze zu verstehen. Bei der Clusteranalyse kann es sich beispielsweise um eine Dichte-Clusteranalyse handeln. Hier können gegebene Ähnlichkeitsstrukturen der Daten durch eine Clusterbildung der Daten im Koordinatensystem - also durch eine Gruppierung der Daten im Koordinatenraum zu Datenclustern - repräsentiert sein. Mit dem Öffnungszustand des Auges kann ausgesagt werden, wie weit das Auge des Insassen geöffnet bzw. geschlossen ist.

Dieses Verfahren kann beispielsweise in Software oder Hardware oder in einer Mischform aus Software und Hardware beispielsweise in einem Steuergerät oder einer Vorrichtung implementiert sein.

Gemäß der Erfindung wird in dem Schritt des Generierens der erste Augenöffnungsdatensatz zumindest einen weiteren Messpunkt aufweisen, der einen nicht durch den Messpunkt repräsentierten ersten Augenöffnungsgrad und/oder eine erste Augenlidbewegungsgeschwindigkeit und/oder eine erste Augenlidbewegungsbeschleunigung des Auges des Insassen in dem ersten Messzeitpunkt repräsentiert. Alternativ oder zusätzlich wird in dem Schritt des Erfassens der zweite Augenöffnungsdatensatz zumindest einen weiteren Erfassungspunkt aufweisen, der einen nicht durch den Erfassungspunkt repräsentierten zweiten Augenöffnungsgrad und/oder eine zweite Augenlidbewegungsgeschwindigkeit und/oder eine zweite Augenlidbewegungsbeschleunigung des Auges des Insassen in dem zweiten Messzeitpunkt repräsentiert. Entsprechend wird in dem Schritt des Ausführens die Clusteranalyse ferner unter Verwendung des weiteren Messpunktes und/oder des weiteren Erfassungspunktes ausgeführt werden. Der weitere Messpunkt bzw. der weitere Erfassungspunkt können vorteilhaft zu einer robusten Erkennung von Augenbewegungsmerkmalen des Insassen beitragen. Hierbei repräsentiert der weitere Messpunkt einen anderen Parameter aus der Menge der Parameter Augenöffnungsgrad, erste Augenlidbewegungsgeschwindigkeit und/oder erste Augenlidbewegungsbeschleunigung des Auges des Insassen als der Messpunkt, sodass die Messpunkte des ersten Augenöffnungsdatensatzes auf Messpunkten basieren, die unterschiedliche physikalische Größen repräsentieren. Analog repräsentiert der weitere Erfassungspunkt einen anderen Parameter aus der Menge der Parameter Augenöffnungsgrad, Augenlidbewegungsgeschwindigkeit und/oder Augenlidbewegungsbeschleunigung des Auges des Insassen als der Erfassungspunkt, sodass die Erfassungspunkte des zweiten Augenöffnungsdatensatzes auf Erfassungspunkten basieren, die unterschiedliche physikalische Größen repräsentieren.

Das Verfahren kann ferner einen Schritt des Mittelns unter Verwendung des Messpunktes und des Erfassungspunktes aufweisen, wenn der erste Augenöffnungsdatensatz und der zweite Augenöffnungsdatensatz dem ersten Datencluster zugeordnet sind. So kann eine erste Kenngröße des ersten Datenclusters gebildet werden, wobei die erste Kenngröße einen Wert für ein Augenöffnungsniveau des Auges des Insassen repräsentieren kann, wenn der Messpunkt und der Erfassungspunkt Augenöffnungsgrade des Auges repräsentieren. Hierbei sollten der Messpunkt und der Erfassungspunkt gleiche physikalische Größen repräsentieren. Unter dem Augenöffnungsniveau ist ein durchschnittlicher Augenöffnungsgrad innerhalb des gleitenden Zeitfensters zu verstehen. Das Augenöffnungsniveau kann in Metern erfasst werden. Gemäß dieser Ausführungsform kann das Augenöffnungsniveau, das für eine Erkennung von Schläfrigkeit oder Sekundenschlaf des Insassen essenziell ist, schnell und robust ermittelt werden.

Gemäß einer Ausführungsform können in dem Schritt des Mittelns der Messpunkt und der Erfassungspunkt gewichtet gemittelt werden, um die erste Kenngröße zu bestimmen. So kann das Augenöffnungsniveau noch zuverlässiger bestimmt werden.

Beispielsweise kann in dem Schritt des Mittelns der Messpunkt in Abhängigkeit von dem Erfassungspunkt gewichtet werden, um die erste Kenngröße zu bestimmen. Insbesondere kann der Messpunkt in Abhängigkeit von einem Wert des Erfassungspunktes gewichtet werden, um die erste Kenngröße zu bestimmen. Hierbei kann der Messpunkt in Abhängigkeit von einem Wert des Erfassungspunktes, speziell in Abhängigkeit von einer Differenz eines Wertes des Messwertes und des Wertes des Erfassungspunktes gewichtet werden. Dabei kann insbesondere eine Größe dieser Differenz zum Gewichten des Messpunktes und/oder des Erfassungspunktes verwendet werden. Mit einer derartigen dichteabhängigen Gewichtung kann vorteilhaft verhindert werden, dass Messfehler und -ungenauigkeiten ein Ergebnis des zu ermittelten Augenöffnungsniveaus verfälschen.

Gemäß einer Ausführungsform kann das Verfahren einen Schritt des Verwerfens des zweiten Augenöffnungsdatensatzes aufweisen, wenn der zweite Augenöffnungsdatensatz dem ersten Datencluster nicht zugeordnet ist. So kann ohne Weiteres Rechenaufwand im Verfahren eingespart werden, speziell wenn der zweite Augenöffnungsdatensatz eventuell durch Messfehler nicht brauchbar ist.

Gemäß einer weiteren Ausführungsform kann in dem Schritt des Ausführens der erste Augenöffnungsdatensatz dem ersten Datencluster zugeordnet werden und der zweite Augenöffnungsdatensatz einem zweiten Datencluster zugeordnet werden, um die Augenöffnungsdaten zu klassieren, wobei der zweite Datencluster einen weiteren Öffnungszustand des Auges des Insassen repräsentiert. Die Zuordnung der Augenöffnungsdatensätze zu verschiedenen Datenclustern ermöglicht die einfache und robuste Erkennung von Augenbewegungsmerkmalen wie beispielsweise Blinzelbewegungen und Tachoblicke des Insassen.

Beispielsweise repräsentiert in dem Schritt des Ausführens der Öffnungszustand ein offenes Auge des Insassen und der weitere Öffnungszustand ein geschlossenes Auge des Insassen.

Gemäß einer weiteren Ausführungsform umfasst das Verfahren einen Schritt des Einlesens eines dritten Augenöffnungsdatensatzes zu einem dritten Messzeitpunkt in dem gleitenden Zeitfenster. Der dritte Augenöffnungsdatensatz kann zumindest einen Ermittlungspunkt aufweisen, der einen dritten Augenöffnungsgrad und/oder eine dritte Augenlidbewegungsgeschwindigkeit und/oder eine dritte Augenlidbewegungsbeschleunigung des Auges des Insassen in dem dritten Messzeitpunkt repräsentiert. Entsprechend kann in dem Schritt des Ausführens die Clusteranalyse unter Verwendung des zumindest einen Ermittlungspunktes des dritten Augenöffnungsdatensatzes ausgeführt werden, um zumindest den dritten Augenöffnungsdatensatz einem dritten Datencluster zuzuordnen, um die Augenöffnungsdaten zu klassieren. Der dritte Datencluster kann einen Übergangszustand des Auges des Insassen repräsentieren. Der Ermittlungspunkt kann einen Wert auf einer Koordinatenachse zur Darstellung dritten Augenöffnungsdatensatzes bilden. Gemäß dieser Ausführungsform kann die Diskriminierung unterschiedlicher Augenbewegungsmerkmale noch weitergehend verbessert werden.

Der Übergangszustand des Auges des Insassen kann eine Öffnungsphase oder eine Schließphase des Auges repräsentieren.

Es wird ferner ein Verfahren zum Erfassen einer Schläfrigkeit und/oder eines Sekundenschlafes eines Insassen eines Fahrzeugs vorgestellt, wobei das Verfahren den folgenden Schritt aufweist:
Ermitteln der Schläfrigkeit und/oder des Sekundenschlafes unter Verwendung von gemäß einem Verfahren gemäß einer der im Vorangegangenen aufgeführten Ausführungsformen klassierten Augenöffnungsdaten.

Der hier vorgestellte Ansatz schafft ferner eine Vorrichtung, die ausgebildet ist, um die Schritte einer Variante eines hier vorgestellten Verfahrens in entsprechenden Einrichtungen durchzuführen, anzusteuern bzw. umzusetzen.

Auch durch diese Ausführungsvariante der Erfindung in Form einer Vorrichtung kann die der Erfindung zugrunde liegende Aufgabe schnell und effizient gelöst werden.

Hierzu kann die Vorrichtung zumindest eine Recheneinheit zum Verarbeiten von Signalen oder Daten, zumindest eine Speichereinheit zum Speichern von Signalen oder Daten, zumindest eine Schnittstelle zu einem Sensor oder einem Aktor zum Einlesen von Sensorsignalen von dem Sensor oder zum Ausgeben von Daten- oder Steuersignalen an den Aktor und/oder zumindest eine Kommunikationsschnittstelle zum Einlesen oder Ausgeben von Daten aufweisen, die in ein Kommunikationsprotokoll eingebettet sind. Die Recheneinheit kann beispielsweise ein Signalprozessor, ein Mikrocontroller oder dergleichen sein, wobei die Speichereinheit ein Flash-Speicher, ein EPROM oder eine magnetische Speichereinheit sein kann. Die Kommunikationsschnittstelle kann ausgebildet sein, um Daten drahtlos und/oder leitungsgebunden einzulesen oder auszugeben, wobei eine Kommunikationsschnittstelle, die leitungsgebundene Daten einlesen oder ausgeben kann, diese Daten beispielsweise elektrisch oder optisch aus einer entsprechenden Datenübertragungsleitung einlesen oder in eine entsprechende Datenübertragungsleitung ausgeben kann.

Unter einer Vorrichtung kann vorliegend ein elektrisches Gerät verstanden werden, das Sensorsignale verarbeitet und in Abhängigkeit davon Steuer- und/oder Datensignale ausgibt. Die Vorrichtung kann eine Schnittstelle aufweisen, die hard- und/oder softwaremäßig ausgebildet sein kann. Bei einer hardwaremäßigen Ausbildung können die Schnittstellen beispielsweise Teil eines sogenannten System-ASICs sein, der verschiedenste Funktionen der Vorrichtung beinhaltet. Es ist jedoch auch möglich, dass die Schnittstellen eigene, integrierte Schaltkreise sind oder zumindest teilweise aus diskreten Bauelementen bestehen. Bei einer softwaremäßigen Ausbildung können die Schnittstellen Softwaremodule sein, die beispielsweise auf einem Mikrocontroller neben anderen Softwaremodulen vorhanden sind.

In einer vorteilhaften Ausgestaltung verwendet die Vorrichtung eine Dichte-Clusteranalyse, um aus einem Augenöffnungssignal Augenbewegungsmerkmale bzw. Augenfeatures zumindest eines Auges des Insassen für die Schläfrigkeitserkennung bzw. die Sekundenschlaferkennung herauszufiltern.

Von Vorteil ist auch ein Computerprogrammprodukt oder Computerprogramm mit Programmcode, der auf einem maschinenlesbaren Träger oder Speichermedium wie einem Halbleiterspeicher, einem Festplattenspeicher oder einem optischen Speicher gespeichert sein kann und zur Durchführung, Umsetzung und/oder Ansteuerung der Schritte eines Verfahrens nach einer der vorstehend beschriebenen Ausführungsformen verwendet wird, insbesondere wenn das Programmprodukt oder Programm auf einem Computer oder einer Vorrichtung ausgeführt wird.

Ausführungsbeispiele der Erfindung sind in den Zeichnungen dargestellt und in der nachfolgenden Beschreibung näher erläutert. Es zeigt:
Fig. 1 eine schematische Darstellung eines Fahrzeugs mit einer Vorrichtung zum Erfassen einer Schläfrigkeit und/oder eines Sekundenschlafes eines Insassen des Fahrzeugs gemäß einem Ausführungsbeispiel;
Fig. 2 ein Blockschaltbild einer Vorrichtung zum Klassieren von Augenöffnungsdaten eines Insassen eines Fahrzeugs gemäß einem Ausführungsbeispiel;
Fig. 3 ein Ablaufdiagramm eines Verfahrens zum Klassieren von Augenöffnungsdaten eines Insassen eines Fahrzeugs gemäß einem Ausführungsbeispiel;
Fig. 4 bis 7 Diagramme zur Darstellung von gemäß dem Verfahren aus Fig. 3 erstellten Datenclustern von Augenöffnungsdaten gemäß einem Ausführungsbeispiel;
Fig. 8 ein Ablaufdiagramm eines Verfahrens zum Erfassen einer Schläfrigkeit und/oder eines Sekundenschlafes eines Insassen eines Fahrzeugs gemäß einem Ausführungsbeispiel; und
Fig. 9 eine Darstellung einer Architektur eines Gesamtsystems zum Überwachen einer Schläfrigkeit und/oder eines Sekundenschlafes eines Insassen eines Fahrzeugs gemäß einem Ausführungsbeispiel.

In der nachfolgenden Beschreibung günstiger Ausführungsbeispiele der vorliegenden Erfindung werden für die in den verschiedenen Figuren dargestellten und ähnlich wirkenden Elemente gleiche oder ähnliche Bezugszeichen verwendet, wobei auf eine wiederholte Beschreibung dieser Elemente verzichtet wird.

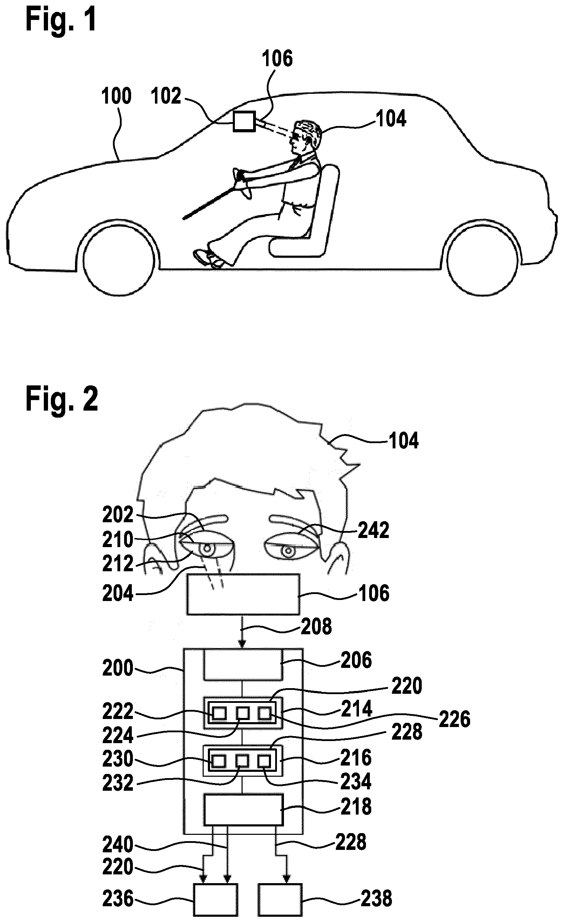

Fig. 1 zeigt eine schematische Darstellung eines Fahrzeugs 100 mit einer Vorrichtung 102 zum Erfassen einer Schläfrigkeit und/oder eines Sekundenschlafes eines Insassen 104 des Fahrzeugs 102 gemäß einem Ausführungsbeispiel. Bei dem Fahrzeug 100 handelt sich um einen Personenkraftwagen. Alternativ kann es sich bei dem Fahrzeug 100 auch um ein anderes straßengebundenes Fahrzeug wie beispielsweise einen Lastkraftwagen handeln. Bei dem Insassen 104 handelt es sich hier um einen Fahrer 104 des Fahrzeugs 100.

Die Vorrichtung 102 ist hierbei mit einer im Fahrzeug 100 installierten Kamera 106 verbunden, die die Augen des Fahrers 104 erfasst. Insbesondere erfasst die Kamera 106 eine Position der Augenlider der Augen des Fahrers 104, beispielsweise einen Abstand zwischen den Augenlidern zumindest eines der Augen und eine Geschwindigkeit bzw. eine Beschleunigung einer Bewegung der Augenlider zumindest eines der Augen. Diese Informationen lassen sich zu Augenöffnungsdaten zusammenfassen, die gemäß dem hier vorgestellten Ansatz klassiert und in der Vorrichtung 102 zur Erkennung eines Schläfrigkeitszustands oder eines Sekundenschlafes des Fahrers 104 des Fahrzeugs 100 verarbeitet werden.

Fig. 2 zeigt ein Blockschaltbild einer Vorrichtung 200 zum Klassieren von Augenöffnungsdaten eines Insassen 104 eines Fahrzeugs gemäß einem Ausführungsbeispiel. Mittels der Vorrichtung 200 klassierte Augenöffnungsdaten können in der in Fig. 1 gezeigten Vorrichtung zum Erkennen einer Schläfrigkeit bzw. eines Sekundenschlafes verwendet werden. Die Vorrichtung 200 ist Teil der in Fig. 1 gezeigten Vorrichtung zum Erkennen einer Schläfrigkeit bzw. eines Sekundenschlafes oder ist mit dieser gekoppelt bzw. koppelbar. Die Vorrichtung 200 ist ausgebildet, um Augenöffnungsdaten zumindest eines Auges 202 des Insassen 104, hier des rechten Auges 202, zu klassieren.

Die auf die Augen des Insassen 104 gerichtete Kamera 106 empfängt ein Augenöffnungssignal 204 des rechten Auges 202 des Insassen 104. Das Augenöffnungssignal 204 umfasst Messungen von Bewegungen der Augenlider des Auges 202 über eine Mehrzahl von Messzeitpunkten hinweg. Das Augenöffnungssignal 204 repräsentiert damit einen Verlauf der Augenöffnungsdaten über mehrere Zeitpunkte hinweg.

Über eine geeignete Schnittstelle liest eine Einleseeinrichtung 206 der Vorrichtung 200 das Augenöffnungssignal 204 bildende Augenöffnungsdaten 208 in die Vorrichtung 200 ein. Die Augenöffnungsdaten 208 umfassen Informationen zu einem Augenöffnungsgrad, einer Geschwindigkeit sowie einer Beschleunigung der Augenlider des Auges 202 zu einem Messzeitpunkt während der Erfassung des Auges 202. Die Augenöffnungsdaten 208 bzw. das Augenöffnungssignal 204 werden in einem gleitenden Zeitfenster von der Kamera 106 oder einer mit der Kamera 106 gekoppelten Einrichtung erfasst.

Unter dem Augenöffnungsgrad ist ein Abstand von einem oberen Augenlid 210 zu einem unteren Augenlid 212 des Auges 202 zu einem Messzeitpunkt zu verstehen. Der Augenöffnungsgrad wird in Metern erfasst. Die Geschwindigkeit sowie die Beschleunigung der Augenlider 210, 212 wird in vollständigen oder unvollständigen Öffnungs- bzw. Schließphasen der Augenlider 210, 212 erfasst.

Die Vorrichtung 200 weist neben der Einleseeinrichtung 206 eine Generiereinrichtung 214, eine Erfassungseinrichtung 216 und eine Ausführeinrichtung 218 auf.

Die Generiereinrichtung 214 ist ausgebildet, um zu einem ersten Messzeitpunkt in dem gleitenden Zeitfenster basierend auf den Augenöffnungsdaten 208 einen ersten Augenöffnungsdatensatz 220 zu generieren. Der erste Augenöffnungsdatensatz 220 weist gemäß einem Ausführungsbeispiel einen Messpunkt 222, einen weiteren Messpunkt 224 und einen zweiten weiteren Messpunkt 226 auf. Beispielsweise repräsentiert der Messpunkt 222 einen ersten Augenöffnungsgrad des Auges 202 in dem ersten Messzeitpunkt, der weitere Messpunkt 224 eine Augenlidbewegungsgeschwindigkeit zumindest eines der Augenlider 210, 212 des Insassen 104 in dem ersten Messzeitpunkt und der zweite weitere Messpunkt 226 eine Augenlidbewegungsbeschleunigung des Auges 202 des Insassen 104 in dem ersten Messzeitpunkt.

Die Erfassungseinrichtung 216 ist ausgebildet, um zu einem zweiten Messzeitpunkt in dem gleitenden Zeitfenster basierend auf den Augenöffnungsdaten 208 einen zweiten Augenöffnungsdatensatz 228 zu erfassen. Der zweite Augenöffnungsdatensatz 228 weist gemäß einem Ausführungsbeispiel einen Erfassungspunkt 230, einen weiteren Erfassungspunkt 232 und einen zweiten weiteren Erfassungspunkt 234 auf. Beispielsweise repräsentiert der Erfassungspunkt 230 einen zweiten Augenöffnungsgrad des Auges 202 in dem zweiten Messzeitpunkt, der weitere Erfassungspunkt 232 eine Augenlidbewegungsgeschwindigkeit zumindest eines der Augenlider 210, 212 des Insassen 104 in dem zweiten Messzeitpunkt und der zweite weitere Erfassungspunkt 234 eine Augenlidbewegungsbeschleunigung des Auges 202 des Insassen 104 in dem zweiten Messzeitpunkt.

Es können gemäß Ausführungsbeispielen mehr oder weniger als die je drei Messpunkte 222, 224, 226 und Erfassungspunkte 230, 232, 234 zur Bildung der Augenöffnungsdatensätze 220, 228 vorliegen. Die Augenöffnungsdatensätze 220, 228 können je nach Ausführungsbeispiel der Vorrichtung 200 aus unterschiedlichen Kombinationen aus Augenöffnungsgrad, Augenlidbewegungsgeschwindigkeit und Augenlidbewegungsbeschleunigung des Auges 202 gebildet werden. Es können auch nur Teile dieser Informationen in den Augenöffnungsdatensätzen 220, 228 enthalten sein.

Die Ausführeinrichtung 218 ist ausgebildet, um unter Verwendung der Messpunkte 222, 224, 226 und Erfassungspunkte 230, 232, 234 unter Einsatz geeigneter Algorithmen eine Clusteranalyse der Augenöffnungsdatensätze 220, 228 auszuführen. Entsprechend einem Ergebnis der Clusteranalyse ordnet die Ausführeinrichtung 218 den ersten Augenöffnungsdatensatz 220 einem ersten Datencluster 236 aus einer Mehrzahl von Datenclustern zu und den zweiten Augenöffnungsdatensatz 228 einem zweiten Datencluster 238 aus der Mehrzahl von Datenclustern zu. Gemäß einem Ausführungsbeispiel können auch beide Augenöffnungsdatensätze 220, 228 z. B. dem ersten Datencluster 236 zugeordnet werden. Dies ist beispielsweise der Fall, wenn der erste Messzeitpunkt 222 und der zweite Messzeitpunkt 224 zeitlich sehr nahe beieinander liegen und/oder der erste Erfassungspunkt 230 und der zweite Erfassungspunkt 232 zeitlich sehr nahe beieinander liegen.

Gemäß einem alternativen Ausführungsbeispiel kann die Ausführeinrichtung 218 ausgebildet sein, um den zweiten Augenöffnungsdatensatz 228 zu verwerfen, wenn der zweite Augenöffnungsdatensatz 228 nicht dem ersten Datencluster 236 zugeordnet wird.

Mit dem Zuordnen der Augenöffnungsdatensätze 220, 228 zu Datenclustern 236, 238 werden die Augenöffnungsdatensätze 220, 228 klassiert, also einem von mehreren Öffnungszuständen oder Übergangszuständen des von der Kamera 106 erfassten Auges 202 zugeordnet. Beispielsweise definiert der erste Datencluster 236 einen möglichen ersten Öffnungszustand des Auges 202, in dem das Auge 202 offen ist, und der zweite Datencluster 238 einen möglichen zweiten Öffnungszustand des Auges 202, in dem das Auge 202 geschlossen ist. Gemäß Ausführungsbeispielen kann ein dritter Datencluster einen möglichen ersten Übergangszustand des Auges 202 definieren, in dem das Auge 202 sich in einer Öffnungsphase befindet, und ein vierter Datencluster einen möglichen zweiten Übergangszustand des Auges 202 definieren, in dem das Auge 202 sich in einer Schließphase befindet.

Selbstverständlich kann die Vorrichtung 200 ausgebildet sein, um unter Verwendung ihrer Einrichtungen 206, 214, 216, 218 auch mehr als die zwei Datensätze 220, 228 zu verarbeiten und einem oder mehreren Datenclustern 236, 238 zuzuordnen.

Gemäß einem Ausführungsbeispiel des hierin vorgestellten Konzepts ist die Ausführeinrichtung 218 oder eine andere Einrichtung der Vorrichtung 200 ausgebildet, um - für den Fall, dass der erste Augenöffnungsdatensatz 220 und der zweite Augenöffnungsdatensatz 228 dem ersten Datencluster 236 zugeordnet sind, und den Fall, dass der Messpunkt 222 und der Erfassungspunkt 230 jeweils einen Augenöffnungsgrad repräsentieren - unter Verwendung zumindest eines der Messpunkte 222, 224, 226 und der Erfassungspunkte 230, 232, 234 die Augenöffnungsdatensätze 220, 228 einem Mittelungsprozess zu unterwerfen. So wird eine Kenngröße 240 für den ersten Datencluster 236 gebildet, die einen sehr robusten Wert für ein Augenöffnungsniveau des Auges 202 des Insassen 104 repräsentiert.

Das Augenöffnungsniveau - kurz AON bzw. EON für die englische Bezeichnung "Eye Opening Niveau" - ist ein Wert für den durchschnittlichen Augenöffnungsgrad innerhalb eines Zeitfensters, wobei Zeitpunkte, in denen das Auge nicht voll geöffnet ist, wie beispielsweise Blinzler bzw. Blinzelereignisse oder Tachoblicke, außer Acht gelassen werden. Das Augenöffnungsniveau wird in Metern erfasst und ist essenziell für eine möglichst fehlerfreie Erkennung der Schläfrigkeit oder des Sekundenschlafs durch die in Fig. 1 gezeigte Vorrichtung.

Gemäß einem Ausführungsbeispiel kann es sich bei dem in der der Vorrichtung 200 ausgeführten Mittelungsprozess zur Bestimmung der Kenngröße 240 für den ersten Datencluster 236 um einen gewichteten Mittelungsprozess handeln.

Analog zu dem oben beschriebenen Vorgehen können in der Vorrichtung 200 für alle vorhandenen Datencluster 236, 238 ihnen zugeordnete Kenngrößen berechnet werden.

Für den beispielhaften Fall, dass die Ausführeinrichtung 218 eine Dichte-Clusteranalyse ausführt, kann in dem gewichteten Mittelungsprozess der Messpunkt 222 bzw. die weiteren Messpunkte 224, 226 in Abhängigkeit von dem Erfassungspunkt 230 bzw. den weiteren Erfassungspunkten 232, 234 gewichtet werden. Besonders vorteilhaft ist es, wenn der oder die Messpunkte 222, 224, 226 in Abhängigkeit von einer Nähe zu dem oder den Erfassungspunkten 230, 232, 234 gewichtet werden, um die Kenngröße 240 zu bestimmen. Selbstverständlich kann die Vorrichtung 200 analog zu dem oben erläuterten Vorgehen zusätzlich oder alternativ zeitgleich oder zeitlich versetzt zu den Berechnungen für das rechte Auge 202 Augenöffnungsdaten des linken Auges 242 des Insassen 104 klassieren, um eine Schläfrigkeits- oder einen Sekundenschlafanalyse des Insassen 104 vorzubereiten bzw. zu unterstützen.

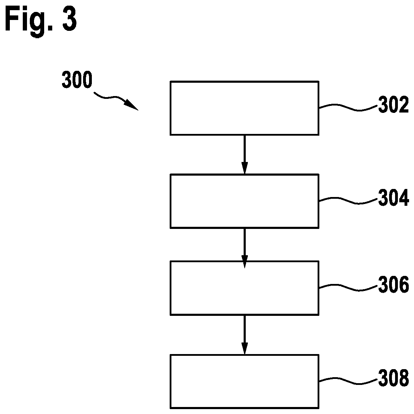

Fig. 3 zeigt ein Ausführungsbeispiel eines Ablaufdiagramms eines Verfahrens 300 zum Klassieren von Augenöffnungsdaten eines Insassen eines Fahrzeugs. Das Verfahren 300 kann von der in Fig. 2 gezeigten Vorrichtung zum Klassieren bzw. ihren Einrichtungen ausgeführt werden.

In einem Schritt des Generierens 302 wird zu einem ersten Messzeitpunkt in einem gleitenden Zeitfenster ein zumindest einen Messpunkt aufweisender erster Augenöffnungsdatensatz generiert. In einem Schritt des Erfassens 304 wird zu einem zweiten Messzeitpunkt in dem gleitenden Zeitfenster ein zumindest einen Erfassungspunkt aufweisender zweiter Augenöffnungsdatensatz erfasst. In einem Schritt des Ausführens 306 wird unter Verwendung des Messpunktes und des Erfassungspunktes eine Clusteranalyse ausgeführt, um den ersten Augenöffnungsdatensatz und/oder zweiten Augenöffnungsdatensatz einem ersten Datencluster zuzuordnen, um die Augenöffnungsdaten zu klassieren.

Gemäß einem Ausführungsbeispiel weist das Verfahren 300 einen Schritt des Mittelns 308 des ersten Augenöffnungsdatensatzes und des zweiten Augenöffnungsdatensatzes unter Verwendung des Messpunktes und des Erfassungspunktes auf, um eine erste Kenngröße des ersten Datenclusters zu bilden.

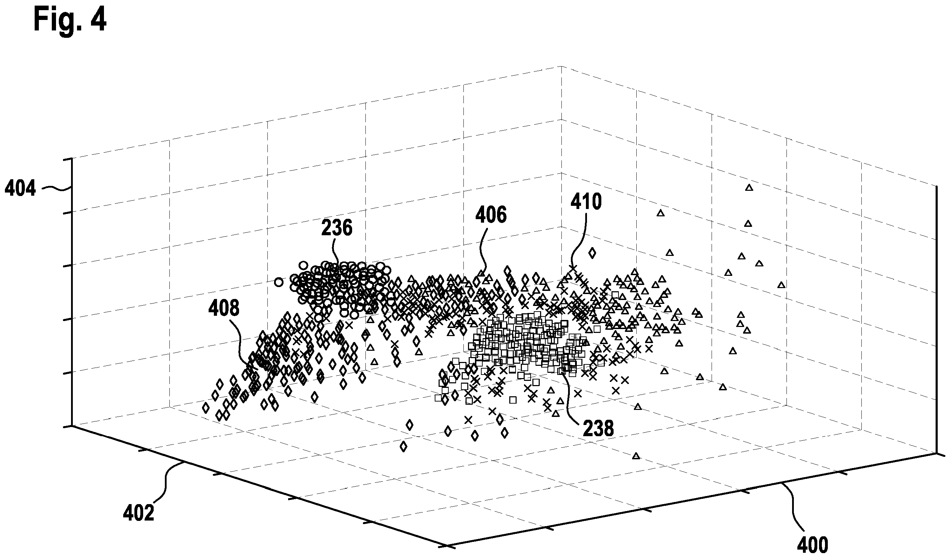

Figuren 4 bis 7 zeigen Diagramme zur Darstellung von gemäß dem Berechnungsverfahren aus Fig. 3 erstellten Datenclustern von Augenöffnungsdaten gemäß einem Ausführungsbeispiel. Gezeigt ist jeweils ein Koordinatensystem, in dem mehrere gemäß dem Berechnungsverfahren ermittelte Datencluster schemenhaft als Wolke dargestellt sind. Allen Diagrammen in den Figuren 4 bis 7 ist eine einzige Berechnung zugrunde gelegt, die die Diagramme aus unterschiedlichen Blickwinkeln zeigen.

Fig. 4 zeigt ein 3-dimensionales kartesisches Koordinatensystem. Auf der Abszisse 400 ist eine Augenlidbewegungsgeschwindigkeit in Metern pro Sekunde (m/s) aufgetragen. Auf der Ordinate 402 ist der Augenöffnungsgrad in Metern (m) aufgetragen. Auf der Applikate 404 ist eine Augenlidbewegungsbeschleunigung in Metern pro Sekunde im Quadrat (m/s²) aufgetragen. Das Koordinatensystem in Fig. 4 zeigt eine Vielzahl von gemäß dem Berechnungsverfahren erstellten bzw. zugeordneten Datensätzen von Augenöffnungsdaten eines Fahrzeuginsassen. Viele der Augenöffnungsdatensätze sind zu Datenclustern gruppiert, die durch das Auge des Betrachters klar voneinander unterscheidbar sind.

Jeder Datencluster repräsentiert in Form einer Datenwolke in der Darstellung in Fig. 4 eine von mehreren Kategorien unterschiedlicher Zustände der Augen bzw. der Augenlider des betrachteten Fahrzeuginsassen. So repräsentiert der bereits im Zusammenhang mit Fig. 2 erläuterte erste Datencluster 236, der aus einer Gruppierung einer Mehrzahl von mittels Kreisen symbolisierten Datensätzen gebildet ist, einen ersten Öffnungszustand des Auges oder der Augen des Fahrzeuginsassen, in dem das Auge oder die Augen offen ist bzw. sind.

Der ebenfalls bereits im Zusammenhang mit Fig. 2 erläuterte zweite Datencluster 238, der aus einer Gruppierung einer Mehrzahl von mittels Quadraten symbolisierten Datensätzen gebildet ist, repräsentiert einen zweiten Öffnungszustand des Auges oder der Augen des Fahrzeuginsassen, in dem das Auge oder die Augen geschlossen ist bzw. sind.

Ein dritter Datencluster 406, der aus einer Gruppierung einer Mehrzahl von mittels Dreiecken symbolisierten Datensätzen gebildet ist, repräsentiert einen ersten Übergangszustand des Auges oder der Augen des Fahrzeuginsassen, in dem das Auge oder die Augen sich in einer Öffnungsphase befindet bzw. befinden.

Ein vierter Datencluster 408, der aus einer Gruppierung einer Mehrzahl von mittels Rauten symbolisierten Datensätzen gebildet ist, repräsentiert einen zweiten Übergangszustand des Auges oder der Augen des Fahrzeuginsassen, in dem das Auge oder die Augen sich in einer Schließphase befindet bzw. befinden. Datensätze 410, die Ausreißer der erfassten Augenöffnungsdaten repräsentieren, sind mittels Kreuzen in dem Koordinatensystem in Fig. 4 eingetragen.

Wie die Darstellung in Fig. 4 zeigt, sind die einzelnen Datencluster 236, 238, 406, 408 durch eine unterschiedliche individuelle Dichte und einen unterschiedlichen individuellen Verteilungsbereich im Koordinatensystem gekennzeichnet. So sind die Datencluster 236 und 238 besonders dicht und räumlich eng begrenzt. Die Datencluster 406 und 408 hingegen weisen eine geringere Dichte auf und erstrecken sich in einem größeren dreidimensionalen Raum, der weniger eindeutig begrenzt ist als bei den Datenclustern 236 und 238. Da die einzelnen Datencluster 236, 238, 406, 408 leicht voneinander zu unterscheiden sind und besonders die Ausreißerwerte 410 ohne Weiteres identifiziert werden können, ist eine robuste Trennung und Zuordnung der Augen- bzw. Augenöffnungszustände des Fahrzeuginsassen für eine von Fehlmessungen relativ unbeeinträchtigte Schläfrigkeits- bzw. Sekundenschlaferkennung möglich.

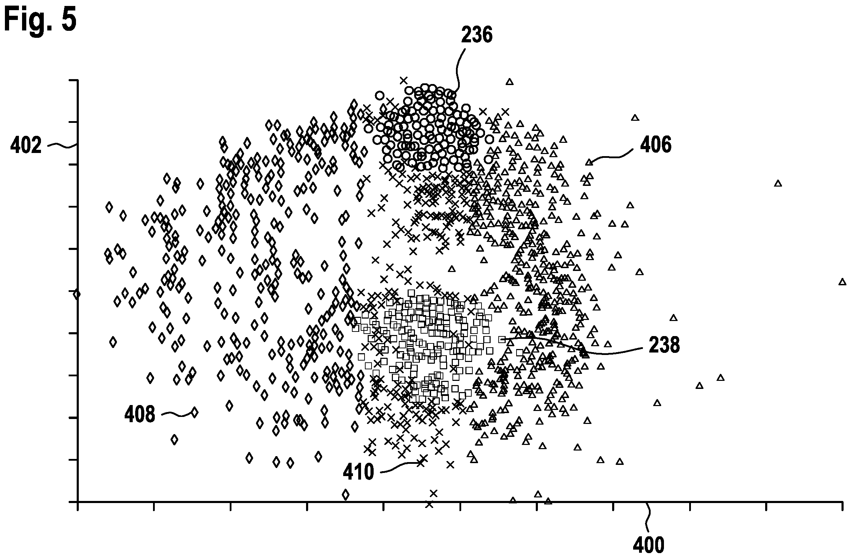

Fig. 5 zeigt die in dem beispielhaften Berechnungsverfahren ermittelten Datencluster 236, 238, 406, 408 in einer zweidimensionalen Ansicht mit Blick auf die Abszisse 400 und die Ordinate 402 des Koordinatensystems aus Fig. 4. Hier ist gut die räumliche Trennung zwischen den Datenclustern 236, 238, 406, 408 zu sehen.

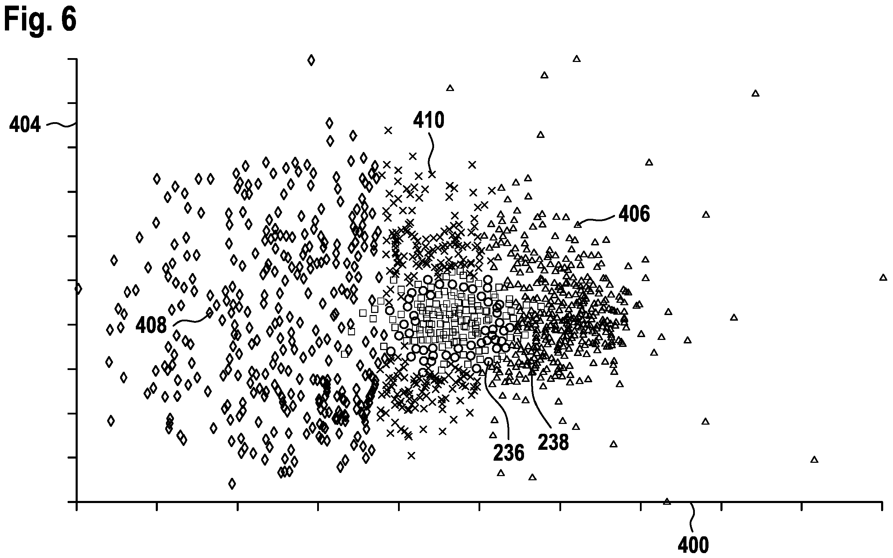

Fig. 6 zeigt die in dem beispielhaften Berechnungsverfahren ermittelten Datencluster 236, 238, 406, 408 in einer weiteren zweidimensionalen Ansicht mit Blick auf die Abszisse 400 und die Applikate 404 des Koordinatensystems aus Fig. 4. In dieser Ansicht überlagert der die geschlossenen Augen repräsentierende zweite Datencluster 238 den in etwa gleich großen und gleich geformten, die offenen Augen repräsentierenden, ersten Datencluster 236. Ferner ist zu sehen, dass der die sich schließenden Augen repräsentierende vierte Datencluster 408 wesentlich weiträumiger gestreut ist als der die sich öffnenden Augen repräsentierende dritte Datencluster 406.

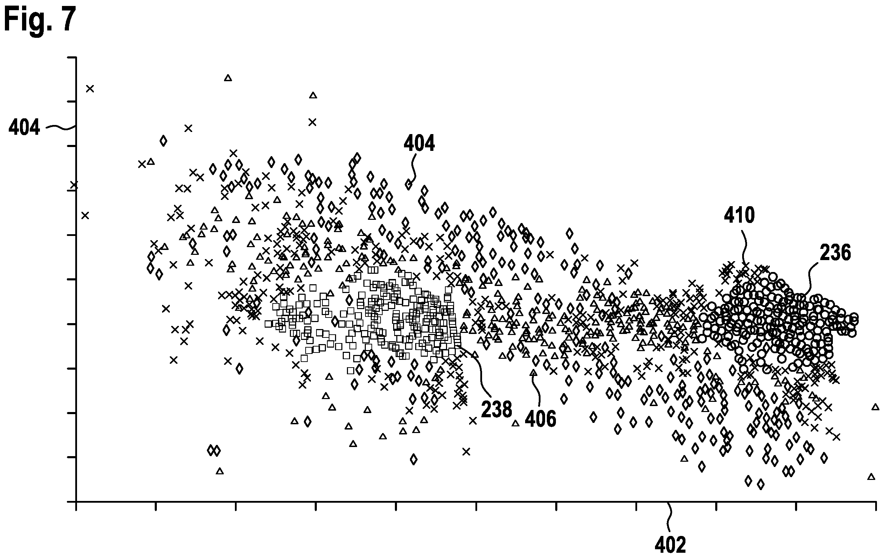

Fig. 7 zeigt die in dem beispielhaften Berechnungsverfahren ermittelten Datencluster 236, 238, 406, 408 in einer weiteren zweidimensionalen Ansicht mit Blick auf die Ordinate 402 und die Applikate 404 des Koordinatensystems aus Fig. 4. Auch hier ist gut die große Dichte der Datencluster 236 und 238 zu erkennen.

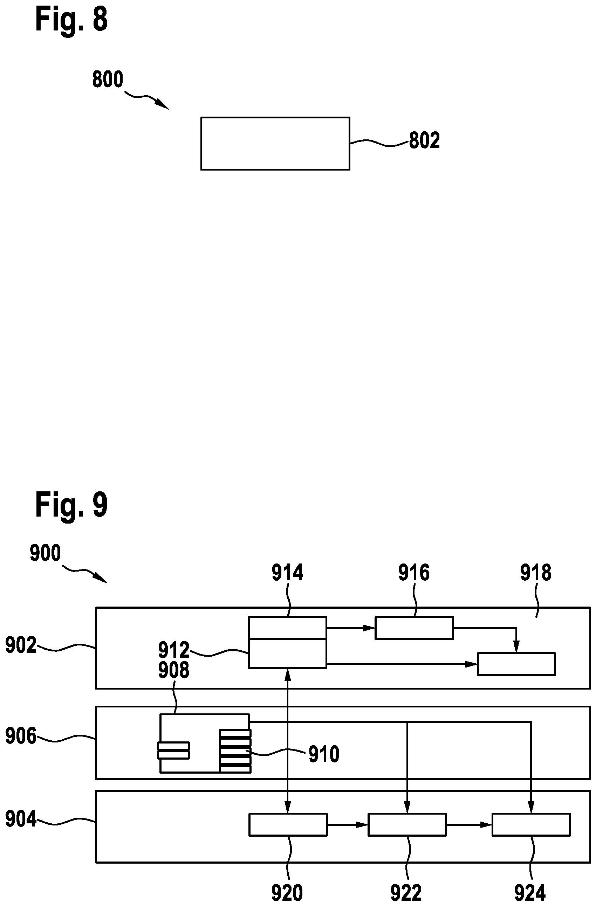

Das anhand der Figuren 4 bis 7 dargestellte und hier vorgeschlagene neuartige Berechnungsverfahren ist im Folgenden anhand eines Ausführungsbeispiels nochmals anschaulich zusammengefasst. Zunächst werden in einem gleitenden Zeitfenster mit einer Dauer von z. B. fünf Minuten für jeden Messzeitpunkt in dem Zeitfenster die Augenöffnungsdaten erfasst. Gemäß einem Ausführungsbeispiel können die Augenöffnungsdaten in einer in der nachfolgenden Fig. 9 genauer erläuterten Augenschlussvorverarbeitungseinheit bzw. ECP-Einheit verarbeitet werden.

Anschließend werden aus den Augenöffnungsdaten gebildete Augenöffnungsdatensätze bzw. deren Messpunkte oder Erfassungspunkte aus dem oben beschriebenen gleitenden Zeitfenster einer Dichte-Clusteranalyse unterzogen. Dabei wird der Raum, in dem sich die Datensätze befinden, nach Regionen durchsucht, die eine hohe Dichte an Datensätzen aufweisen. Alle Datensätze einer solchen Region werden einem gemeinsamen Cluster 236, 238, 406 oder 408 zugeordnet. Eine mögliche Implementierung der Dichte-Clusteranalyse ist der OPTICS-Algorithmus (vgl. Ankerst, Mihael, et al. "OPTICS: ordering points to identify the dustering structure." ACM Sigmod Record. Vol. 28. No. 2. ACM, 1999).

Es lassen sich mehrere dieser Cluster 236, 238, 406 oder 408 identifizieren, was eine Einordnung der zugehörigen Datensätze in unterschiedliche Kategorien zulässt. So kennzeichnet der erste Datencluster 236 eine erste Kategorie, in der die Augen des Fahrzeuginsassen geöffnet sind. Der zweite Datencluster 238 kennzeichnet eine zweite Kategorie, in der die Augen des Fahrzeuginsassen geschlossen sind. Der dritte Datencluster 406 kennzeichnet eine dritte Kategorie, in der sich die Augen des Fahrzeuginsassen in der Öffnungsphase befinden. Der vierte Datencluster 408 kennzeichnet eine vierte Kategorie, in der sich die Augen des Fahrzeuginsassen in der Schließphase befinden. Die Ausreißer 410 bei den Messungen sind ebenfalls bildlich dargestellt. Eine Darstellung weiterer Cluster, die auf weiteren Mess- und Erfassungspunkten basieren, ist selbstverständlich möglich. Beispielsweise kann ein weiterer Cluster Blicke des Fahrzeuginsassen auf das Tachometer kennzeichnen.

In einer bereits erwähnten Weiterbildung des hier vorgeschlagenen Konzepts wird für jeden der Datencluster 236, 238, 406, 408 eine Kenngröße bestimmt. Bei der Bestimmung kann ein Zentrum des jeweiligen Datenclusters 236, 238, 406, 408 betrachtet werden, um die Kenngröße aus einem Mittelwert aller Datensätze innerhalb des Clusters 236, 238, 406, 408 zu bestimmen. Alternativ kann die Kenngröße basierend auf einer Schwerpunktbestimmung ermittelt werden. Hier wird der der gewichtete Mittelwert aller Datensätze innerhalb eines Clusters 236, 238, 406, 408 zugrunde gelegt. Die Gewichtung für jeden Datensatz ist gegeben durch die Dichte der Region, in der er sich befindet.

Da diese Kenngrößen im selben dreidimensionalen Raum wie die zugrunde liegenden Datensätze liegen, lassen sie sich in Werte für den Augenöffnungsgrad, die Bewegungsgeschwindigkeit und die Bewegungsbeschleunigung der Augenlider des Insassen zerlegen. Aus den Kenngrößen der Cluster 236, 238, 406, 408 lässt sich also ein Wert für den Augenöffnungsgrad ablesen, welcher als Augenöffnungsniveau- bzw. EON-Wert für die Schläfrigkeits- bzw. Sekundenschlaferkennung genutzt werden kann. Dieser EON-Wert ist deutlich stabiler, das bedeutet mit weniger Schwankungen behaftet, als ein mit herkömmlichen Methoden bestimmter EON-Wert, da Blinzelereignisse, Tachoblicke usw. nicht in die Berechnung eingehen. Neben dem robusteren EON-Wert ist ein weiterer Vorteil des hier vorgeschlagenen Verfahrens, dass die Informationen der verschiedenen Cluster 236, 238, 406, 408 genutzt werden können, um Blinzelereignisse, Tachoblicke usw. in dem Augenöffnungssignal zu finden.

Fig. 8 zeigt ein Ablaufdiagramm eines Ausführungsbeispiels eines Verfahrens 800 zum Erfassen einer Schläfrigkeit und/oder eines Sekundenschlafes eines Insassen eines Fahrzeugs. In einem Schritt des Ermittelns 802 wird eine Schläfrigkeit und/oder ein Sekundenschlaf des Insassen unter Verwendung von Augenöffnungsdaten, die gemäß dem oben beschriebenen Verfahren zum Klassieren von Augenöffnungsdaten klassiert wurden, ermittelt.

Fig. 9 zeigt eine Darstellung einer Architektur eines Gesamtsystems 900 zum Überwachen eines eines Fahrers eines Fahrzeugs in Bezug auf Schläfrigkeit und/oder Sekundenschlaf gemäß einem Ausführungsbeispiel.

Das Gesamtsystem 900 weist drei Hauptbestandteile 902, 904 und 906 auf. Der erste Hauptbestandteil 902 wird als Schläfrigkeitsklassifizierung bezeichnet. Der zweite Hauptbestandteil 904 wird als Sekundenschlaferkennung bezeichnet. Der dritte Hauptbestandteil 906 umfasst von der Schläfrigkeitsklassifizierung 902 und der Sekundenschlaferkennung 904 gemeinsam genutzte Module 908 sowie eine Vorrichtung 910 zum Bereitstellen des Augenöffnungsniveaus bzw. EONs. Die Module 908 können als Augenschlussvorverarbeitungseinheit bzw. ECP-Einheit (ECP; engl. Eye Closure Preprocessing) 908 bezeichnet werden. Die Augenschlussvorverarbeitungseinheit 908 umfasst eine Erfassung des Augenschlusses rechts und links, eine Filterung des Augenschlusses, eine Geschwindigkeitserfassung des Augenschlusses, eine Beschleunigungserfassung des Augenschlusses, die Vorrichtung 910 für die Bereitstellung des EONs und eine Validierung.

Die Augenschlussvorverarbeitungseinheit 908 gibt einen momentanen Augenschluss, eine Augenschlussgeschwindigkeit und das EON aus.

In der Schläfrigkeitsklassifizierung 902 werden diese Werte in einer Blinzelereignisdetektion 912 verwendet und Blinzelereignisse an eine Blinzelmerkmalsberechnung 914 weitergeleitet.

Die Blinzelmerkmalsberechnung 914 gibt Blinzelmerkmale an eine persönliche Blinzelmerkmalserkennung 916 und ein Modul 918 zur Schläfrigkeitsklassifizierung aus. Das Modul 918 liest von der Blinzelmerkmalserkennung 916 ein persönliches Blinzelverhalten ein und gibt ein Schläfrigkeitsniveau aus.

In der Sekundenschlaferkennung 904 werden die Werte in einer persönlichen Augenschlusserkennung 920, einer Augenschlusserkennung 922 und einem Modul 924 zur Sekundenschlaferkennung verwendet.

Die persönliche Augenschlusserkennung 920 gibt ein persönliches Augen-Offen-Niveau und ein persönliches Augen-Geschlossen-Niveau aus. Beide werden von der Augenschlusserkennung 922 verwendet, um einen binären Augen-Offen-Wert für das Modul 924 bereitzustellen. Das Modul 924 gibt Sekundenschlafereignisse aus.

Gemäß einem Ausführungsbeispiel lässt sich das gemäß dem hier vorgestellten Konzept ermittelte robuste EON ohne Weiteres in das Gesamtsystem 900 zur Erkennung von Schläfrigkeit und/oder Sekundenschlaf eingliedern. Alternativ kann die Berechnung des EONs im Rahmen der Augenschlussvorverarbeitung in der ECP-Einheit 908 stattfinden. Das EON wird anschließend im Rahmen der Blinzelereigniserfassung und der Blinzelmerkmalsberechnung verwendet.

Wie bereits erläutert, gibt das EON an, wie groß ein durchschnittlicher aktueller Abstand der Augenlider im geöffneten Zustand ist. Bei der Berechnung des EONs ist es wichtig, dass mögliche Blinzelereignisse, Tachoblicke etc., nicht in den berechneten Wert mit einfließen, da dieser sonst etwa durch die Blinzelfrequenz und -dauer beeinflusst würde. Je höher dann die Frequenz und Dauer der Blinzelereignisse wäre, desto niedriger würde das daraus resultierende EON ausfallen.

Das Vorhandensein des gemäß dem hier vorgestellten Konzept robusten und mit wenigen Schwankungen behafteten EONs bringt mehrere Vorteile mit sich. So kann eine einfache und robuste Detektion von Blinzelereignissen mit Grenzwerten, die sich relativ zum EON definieren, gewährleistet werden. Es ist ferner eine einfache Berechnung von bestimmten Merkmalen der Blinzelereignisse wie z. B. der Blinzelamplitude möglich. Auch ergibt sich die Möglichkeit zur robusten Berechnung eines PERCLOS-Wertes, der sich auf das 90. Perzentil des EONs als maximalen Öffnungsgrad bezieht.

Umfasst ein Ausführungsbeispiel eine "und/oder"-Verknüpfung zwischen einem ersten Merkmal und einem zweiten Merkmal, so ist dies so zu lesen, dass das Ausführungsbeispiel gemäß einer Ausführungsform sowohl das erste Merkmal als auch das zweite Merkmal und gemäß einer weiteren Ausführungsform entweder nur das erste Merkmal oder nur das zweite Merkmal aufweist.

## Patentansprüche

1. Verfahren (300) zum Klassieren von Augenöffnungsdaten (208) zumindest eines Auges (202, 242) eines Insassen (104) eines Fahrzeugs (100) für eine Schläfrigkeitserfassung und/oder Sekundenschlaferfassung des Insassen (104), wobei das Verfahren (300) von einem elektrischen Gerät durchgeführt wird und die folgenden Schritte aufweist:
Generieren (302) eines ersten Augenöffnungsdatensatzes (220) zu einem ersten Messzeitpunkt in einem gleitenden Zeitfenster, wobei der erste Augenöffnungsdatensatz (220) zumindest einen ersten Messpunkt (222) aufweist, der einen ersten Augenöffnungsgrad und/oder eine erste Augenlidbewegungsgeschwindigkeit und/oder eine erste Augenlidbewegungsbeschleunigung des Auges (202, 242) des Insassen (104) in dem ersten Messzeitpunkt repräsentiert;
Erfassen (304) eines zweiten Augenöffnungsdatensatzes (228) zu einem zweiten Messzeitpunkt in dem gleitenden Zeitfenster, wobei der zweite Augenöffnungsdatensatz (228) zumindest einen ersten Erfassungspunkt (230) aufweist, der einen zweiten Augenöffnungsgrad und/oder eine zweite Augenlidbewegungsgeschwindigkeit und/oder eine zweite Augenlidbewegungsbeschleunigung des Auges (202, 242) des Insassen (104) in dem zweiten Messzeitpunkt repräsentiert; und
Ausführen (306) einer Clusteranalyse unter Verwendung des zumindest einen ersten Messpunktes (222) und des zumindest einen ersten Erfassungspunktes (230), um zumindest den ersten Augenöffnungsdatensatz (220) und/oder zweiten Augenöffnungsdatensatz (228) einem ersten Datencluster (236) zuzuordnen, um die Augenöffnungsdaten (208) zu klassieren, wobei der erste Datencluster (236) einen Öffnungszustand des Auges (202, 242) des Insassen (104) repräsentiert,
**dadurch gekennzeichnet, dass**
- in dem Schritt des Generierens (302) der erste Augenöffnungsdatensatz (220) zumindest einen weiteren Messpunkt (224) aufweist, der einen nicht durch den ersten Messpunkt repräsentierten ersten Augenöffnungsgrad und/oder eine erste Augenlidbewegungsgeschwindigkeit und/oder eine erste Augenlidbewegungsbeschleunigung des Auges (202, 242) des Insassen (104) in dem ersten Messzeitpunkt repräsentiert, wobei der weitere Messpunkt (224) einen anderen Parameter aus der Menge der Parameter Augenöffnungsgrad, erste Augenlidbewegungsgeschwindigkeit und/oder erste Augenlidbewegungsbeschleunigung des Auges des Insassen repräsentiert, als der erste Messpunkt (222), und/oder
- in dem Schritt des Erfassens (304) der zweite Augenöffnungsdatensatz (228) zumindest einen weiteren Erfassungspunkt (232) aufweist, der einen nicht durch den ersten Erfassungspunkt repräsentierten zweiten Augenöffnungsgrad und/oder eine zweite Augenlidbewegungsgeschwindigkeit und/oder eine zweite Augenlidbewegungsbeschleunigung des Auges (202, 242) des Insassen (104) in dem zweiten Messzeitpunkt repräsentiert, wobei der weitere Erfassungspunkt (232) einen anderen Parameter aus der Menge der Parameter Augenöffnungsgrad, Augenlidbewegungsgeschwindigkeit und/oder Augenlidbewegungsbeschleunigung des Auges des Insassen repräsentiert, als der erste Erfassungspunkt (230) und
- wobei in dem Schritt des Ausführens (306) die Clusteranalyse ferner unter Verwendung des weiteren Messpunktes (224) und/oder des weiteren Erfassungspunktes (232) ausgeführt wird.

2. Verfahren (300) gemäß Anspruch 1, **gekennzeichnet durch** einen Schritt des Mittelns (308) unter Verwendung des ersten Messpunktes (222) und des ersten Erfassungspunktes (230), wenn der erste Augenöffnungsdatensatz (220) und der zweite Augenöffnungsdatensatz (228) dem ersten Datencluster (236) zugeordnet sind, um eine erste Kenngröße (240) des ersten Datenclusters (236) zu bilden, wobei die erste Kenngröße (240) einen Wert für ein Augenöffnungsniveau des Auges (202, 242) des Insassen (104) repräsentiert.

3. Verfahren (300) gemäß Anspruch 2, **dadurch gekennzeichnet, dass** in dem Schritt des Mittelns (308) der I erste Messpunkt (222) und der erste Erfassungspunkt (230) gewichtet gemittelt werden, um die erste Kenngröße (240) zu bestimmen.

4. Verfahren (300) gemäß Anspruch 3, **dadurch gekennzeichnet, dass** in dem Schritt des Mittelns (308) der erste Messpunkt (222) in Abhängigkeit von dem I ersten Erfassungspunkt (230) gewichtet wird, insbesondere, wobei der erste Messpunkt (222) in Abhängigkeit von einem Wert des ersten Erfassungspunktes (230) gewichtet wird, um die erste Kenngröße (240) zu bestimmen.

5. Verfahren (300) gemäß einem der vorangegangenen Ansprüche, **gekennzeichnet durch** einen Schritt des Verwerfens des zweiten Augenöffnungsdatensatzes (228), wenn der zweite Augenöffnungsdatensatz (228) dem ersten Datencluster (236) nicht zugeordnet ist.

6. Verfahren (300) gemäß einem der vorangegangenen Ansprüche, **dadurch gekennzeichnet, dass** in dem Schritt des Ausführens (306) der erste Augenöffnungsdatensatz (220) dem ersten Datencluster (236) zugeordnet wird und der zweite Augenöffnungsdatensatz (228) einem zweiten Datencluster (238) zugeordnet wird, um die Augenöffnungsdaten (208) zu klassieren, wobei der zweite Datencluster (238) einen weiteren Öffnungszustand des Auges (202, 242) des Insassen (104) repräsentiert.

7. Verfahren (300) gemäß Anspruch 6, **dadurch gekennzeichnet, dass** in dem Schritt des Ausführens (306) der Öffnungszustand ein offenes Auge (202, 242) des Insassen (104) repräsentiert und der weitere Öffnungszustand ein geschlossenes Auge (202, 242) des Insassen (104) repräsentiert.

8. Verfahren (300) gemäß einem der vorangegangenen Ansprüche, **gekennzeichnet durch** einen Schritt des Einlesens eines dritten Augenöffnungsdatensatzes zu einem dritten Messzeitpunkt in dem gleitenden Zeitfenster, wobei der dritte Augenöffnungsdatensatz zumindest einen Ermittlungspunkt aufweist, der einen dritten Augenöffnungsgrad und/oder eine dritte Augenlidbewegungsgeschwindigkeit und/oder eine dritte Augenlidbewegungsbeschleunigung des Auges (202, 242) des Insassen (104) in dem dritten Messzeitpunkt repräsentiert, wobei in dem Schritt des Ausführens (306) die Clusteranalyse unter Verwendung des zumindest einen Ermittlungspunktes des dritten Augenöffnungsdatensatzes ausgeführt wird, um zumindest den dritten Augenöffnungsdatensatz einem dritten Datencluster (406) zuzuordnen, um die Augenöffnungsdaten (208) zu klassieren, wobei der dritte Datencluster (406) einen Übergangszustand des Auges (202, 242) des Insassen (104) repräsentiert.

9. Verfahren (300) gemäß Anspruch 8, **dadurch gekennzeichnet, dass** der Übergangszustand eine Öffnungsphase des Auges (202, 242) des Insassen (104) oder eine Schließphase des Auges (202, 242) des Insassen (104) repräsentiert.

10. Verfahren (800) zum Erfassen einer Schläfrigkeit und/oder eines Sekundenschlafes eines Insassen (104) eines Fahrzeugs (100), wobei das Verfahren (800) den folgenden Schritt aufweist:
Ermitteln (802) der Schläfrigkeit und/oder des Sekundenschlafes unter Verwendung von gemäß einem Verfahren (300) gemäß einem der vorangegangenen Ansprüche klassierten Augenöffnungsdaten (208).

11. Vorrichtung (102; 200), die eingerichtet ist, um die Schritte eines Verfahrens (300; 800) gemäß einem der vorangegangenen Ansprüche in entsprechenden Einheiten auszuführen.

12. Computerprogramm, das dazu eingerichtet ist, ein Verfahren (300; 800) gemäß einem der vorangegangenen Ansprüche auszuführen.

13. Maschinenlesbares Speichermedium, auf dem das Computerprogramm nach Anspruch 12 gespeichert ist.

## Claims

1. Method (300) for classifying eye opening data (208) of at least one eye (202, 242) of an occupant (104) of a vehicle (100) for detecting drowsiness and/or detecting microsleep of the occupant (104), the method (300) being carried out by an electrical device and having the following steps:
generating (302) a first eye opening data record (220) at a first measurement time in a sliding time window, the first eye opening data record (220) having at least one first measurement point (222) representing a first eye opening degree and/or a first eyelid movement speed and/or a first eyelid movement acceleration of the eye (202, 242) of the occupant (104) at the first measurement time;
capturing (304) a second eye opening data record (228) at a second measurement time in the sliding time window, the second eye opening data record (228) having at least one first capture point (230) representing a second eye opening degree and/or a second eyelid movement speed and/or a second eyelid movement acceleration of the eye (202, 242) of the occupant (104) at the second measurement time; and
executing (306) a cluster analysis using the at least one first measurement point (222) and the at least one first capture point (230) in order to assign at least the first eye opening data record (220) and/or second eye opening data record (228) to a first data cluster (236) in order to classify the eye opening data (208), the first data cluster (236) representing an opening state of the eye (202, 242) of the occupant (104),
**characterized in that**
- in the step of generating (302), the first eye opening data record (220) has at least one further measurement point (224) representing a first eye opening degree not represented by the first measurement point and/or a first eyelid movement speed and/or a first eyelid movement acceleration of the eye (202, 242) of the occupant (104) at the first measurement time, the further measurement point (224) representing, by comparison with the first measurement point (222), a different parameter from the set of parameters: eye opening degree, first eyelid movement speed and/or first eyelid movement acceleration of the eye of the occupant, and/or
- in the step of capturing (304), the second eye opening data record (228) has at least one further capture point (232) representing a second eye opening degree not represented by the first capture point and/or a second eyelid movement speed and/or a second eyelid movement acceleration of the eye (202, 242) of the occupant (104) at the second measurement time, the further capture point (232) representing, by comparison with the first capture point (230), a different parameter from the set of parameters: eye opening degree, eyelid movement speed and/or eyelid movement acceleration of the eye of the occupant, and
- in the step of executing (306), the cluster analysis furthermore being executed using the further measurement point (224) and/or the further capture point (232).

2. Method (300) according to Claim 1, **characterized by** a step of averaging (308) using the first measurement point (222) and the first capture point (230) if the first eye opening data record (220) and the second eye opening data record (228) are assigned to the first data cluster (236) in order to form a first characteristic variable (240) of the first data cluster (236), the first characteristic variable (240) representing a value for an eye opening niveau of the eye (202, 242) of the occupant (104).

3. Method (300) according to Claim 2, **characterized in that** in the step of averaging (308), the first measurement point (222) and the first capture point (230) are averaged in a weighted manner in order to determine the first characteristic variable (240).

4. Method (300) according to Claim 3, **characterized in that** in the step of averaging (308), the first measurement point (222) is weighted as a function of the first capture point (230), in particular the first measurement point (222) being weighted as a function of a value of the first capture point (230), in order to determine the first characteristic variable (240).

5. Method (300) according to any of the preceding claims, **characterized by** a step of discarding the second eye opening data record (228) if the second eye opening data record (228) is not assigned to the first data cluster (236).

6. Method (300) according to any of the preceding claims, **characterized in that** in the step of executing (306), the first eye opening data record (220) is assigned to the first data cluster (236) and the second eye opening data record (228) is assigned to a second data cluster (238) in order to classify the eye opening data (208), the second data cluster (238) representing a further opening state of the eye (202, 242) of the occupant (104).

7. Method (300) according to Claim 6, **characterized in that** in the step of executing (306), the opening state represents an open eye (202, 242) of the occupant (104) and the further opening state represents a closed eye (202, 242) of the occupant (104).

8. Method (300) according to any of the preceding claims, **characterized by** a step of reading in a third eye opening data record at a third measurement time in the sliding time window, the third eye opening data record having at least one ascertaining point representing a third eye opening degree and/or a third eyelid movement speed and/or a third eyelid movement acceleration of the eye (202, 242) of the occupant (104) at the third measurement time, wherein in the step of executing (306), the cluster analysis is executed using the at least one ascertaining point of the third eye opening data record in order to assign at least the third eye opening data record to a third data cluster (406) in order to classify the eye opening data (208), the third data cluster (406) representing a transition state of the eye (202, 242) of the occupant (104).

9. Method (300) according to Claim 8, **characterized in that** the transition state represents an opening phase of the eye (202, 242) of the occupant (104) or a closing phase of the eye (202, 242) of the occupant (104).

10. Method (800) for detecting drowsiness and/or microsleep of an occupant (104) of a vehicle (100), the method (800) having the following step:
ascertaining (802) drowsiness and/or microsleep using eye opening data (208) classified according to a method (300) according to any of the preceding claims.

11. Apparatus (102; 200) configured to carry out the steps of a method (300; 800) according to any of the preceding claims in corresponding units.

12. Computer program configured to carry out a method (300; 800) according to any of the preceding claims.

13. Machine-readable storage medium on which the computer program according to Claim 12 is stored.

## Revendications

1. Procédé (300) permettant de classifier des données d'ouverture des yeux (208) d'au moins un œil (202, 242) d'un occupant (104) d'un véhicule (100) pour une détection de somnolence et/ou d'une détection d'assoupissement de l'occupant (104), le procédé (300) étant exécuté par un appareil électrique et présentant les étapes suivantes consistant à :
générer (302) un premier jeu de données d'ouverture des yeux (220) à un premier instant de mesure dans une fenêtre de temps mobile, dans lequel le premier jeu de données d'ouverture des yeux (220) présente au moins un premier point de mesure (222) qui représente un premier degré d'ouverture des yeux et/ou une première vitesse de mouvement de paupière et/ou une première accélération de mouvement de paupière de l'œil (202, 242) de l'occupant (104) au premier instant de mesure ;
détecter (304) un deuxième jeu de données d'ouverture des yeux (228) à un deuxième instant de mesure dans la fenêtre de temps mobile, dans lequel le deuxième jeu de données d'ouverture des yeux (228) présente au moins un premier point de détection (230) qui représente un deuxième degré d'ouverture des yeux et/ou une deuxième vitesse de mouvement de paupière et/ou une deuxième accélération de mouvement de paupière de l'œil (202, 242) de l'occupant (104) au deuxième instant de mesure ; et
effectuer (306) une analyse par partitionnement en utilisant ledit au moins un premier point de mesure (222) et ledit au moins un premier point de détection (230) pour attribuer au moins le premier jeu de données d'ouverture des yeux (220) et/ou le deuxième jeu de données d'ouverture des yeux (228) à un premier cluster de données (236) afin de classifier les données d'ouverture des yeux (208), le premier cluster de données (236) représentant un état d'ouverture de l'œil (202, 242) de l'occupant (104),
**caractérisé en ce que**
- à l'étape de génération (302), le premier jeu de données d'ouverture des yeux (220) présente au moins un point de mesure supplémentaire (224) qui représente un premier degré d'ouverture des yeux et/ou une première vitesse de mouvement de paupière et/ou une première accélération de mouvement de paupière de l'œil (202, 242) de l'occupant (104) au premier instant de mesure, non représentés par le premier point de mesure ; dans lequel le point de mesure supplémentaire (224) représente un autre paramètre parmi l'ensemble des paramètres degré d'ouverture des yeux, première vitesse de mouvement de paupière et/ou première accélération de mouvement de paupière de l'œil de l'occupant que le premier point de mesure (222), et/ou
- à l'étape de détection (304), le deuxième jeu de données d'ouverture des yeux (228) présente au moins un point de détection supplémentaire (232) qui représente un deuxième degré d'ouverture des yeux et/ou une deuxième vitesse de mouvement de paupière et/ou une deuxième accélération de mouvement de paupière de l'œil (202, 242) de l'occupant (104) au deuxième instant de mesure, non représentés par le premier point de détection, dans lequel le point de détection supplémentaire (232) représente un autre paramètre parmi l'ensemble des paramètres degré d'ouverture des yeux, vitesse de mouvement de paupière et/ou accélération de mouvement de paupière de l'œil de l'occupant que le premier point de détection (230), et
- dans lequel, à l'étape de l'exécution (306), l'analyse par partitionnement est en outre effectuée en utilisant le point de mesure supplémentaire (224) et/ou le point de détection supplémentaire (232).

2. Procédé (300) selon la revendication 1, **caractérisé par** une étape de calcul de moyenne (308) en utilisant le premier point de mesure (222) et le premier point de détection (230) si le premier jeu données d'ouverture des yeux (220) et le deuxième jeu de données d'ouverture des yeux (228) sont attribués au premier cluster de données (236) afin de former une première grandeur caractéristique (240) du premier cluster de données (236), dans lequel la première grandeur caractéristique (240) représente une valeur pour un niveau d'ouverture des yeux de l'œil (202, 242) de l'occupant (104).

3. Procédé (300) selon la revendication 2, **caractérisé en ce qu'**à l'étape de calcul de moyenne (308), le premier point de mesure (222) et le premier point de détection (230) sont moyennés de manière pondérée afin de déterminer la première grandeur caractéristique (240).

4. Procédé (300) selon la revendication 3, **caractérisé en ce qu'**à l'étape de calcul de moyenne (308), le premier point de mesure (222) est pondéré en fonction du premier point de détection (230), en particulier dans lequel le premier point de mesure (222) est pondéré en fonction d'une valeur du premier point de détection (230) afin de déterminer la première grandeur caractéristique (240).

5. Procédé (300) selon l'une quelconque des revendications précédentes, **caractérisé par** une étape consistant à rejeter le deuxième jeu de données d'ouverture des yeux (228) si le deuxième jeu de données d'ouverture des yeux (228) n'est pas attribué au premier cluster de données (236).

6. Procédé (300) selon l'une quelconque des revendications précédentes, **caractérisé en ce qu'**à l'étape d'exécution (306), le premier jeu de données d'ouverture des yeux (220) est attribué au premier cluster de données (236), et le deuxième jeu de données d'ouverture des yeux (228) est attribué à un deuxième cluster de données (238) afin de classifier les données d'ouverture des yeux (208), le deuxième cluster de données (238) représentant un état d'ouverture supplémentaire de l'œil (202, 242) de l'occupant (104).

7. Procédé (300) selon la revendication 6, **caractérisé en ce qu'**à l'étape d'exécution (306), l'état d'ouverture représente un œil ouvert (202, 242) de l'occupant (104), et l'état d'ouverture supplémentaire représente un œil (202, 242) fermé de l'occupant (104).

8. Procédé (300) selon l'une quelconque des revendications précédentes, **caractérisé par** une étape consistant à lire un troisième jeu de données d'ouverture des yeux à un troisième instant de mesure dans la fenêtre de temps mobile, dans lequel le troisième jeu de données d'ouverture des yeux présente au moins un point de détermination qui représente un troisième degré d'ouverture des yeux et/ou une troisième vitesse de mouvement de paupière et/ou une troisième accélération de mouvement de paupière de l'œil (202, 242) de l'occupant (104) au troisième instant de mesure, dans lequel à l'étape d'exécution (306), l'analyse par partitionnement est effectuée en utilisant ledit au moins un point de détermination du troisième jeu de données d'ouverture des yeux pour attribuer au moins le troisième jeu de données d'ouverture des yeux à un troisième cluster de données (406) afin de classifier les données d'ouverture des yeux (208), le troisième cluster de données (406) représentant un état de transition de l'œil (202, 242) de l'occupant (104).

9. Procédé (300) selon la revendication 8, **caractérisé en ce que** l'état de transition représente une phase d'ouverture de l'œil (202, 242) de l'occupant (104) ou une phase de fermeture de l'œil (202, 242) de l'occupant (104) .

10. Procédé (800) permettant de détecter une somnolence et/ou un assoupissement d'un occupant (104) d'un véhicule (100), le procédé (800) présentant l'étape suivante consistant à :
déterminer (802) la somnolence et/ou l'assoupissement en utilisant des données d'ouverture des yeux (208) classifiées selon un procédé (300) selon l'une quelconque des revendications précédentes.

11. Dispositif (102 ; 200) conçu pour exécuter les étapes d'un procédé (300 ; 800) selon l'une quelconque des revendications précédentes dans des unités correspondantes.

12. Programme informatique conçu pour exécuter un procédé (300 ; 800) selon l'une des revendications précédentes.

13. Support de stockage lisible par machine, sur lequel est enregistré le programme informatique selon la revendication 12.
